# EUROPEAN PATENT SPECIFICATION

(11) **EP 2 948 099 B1**
(45) Date of publication and mention of the grant of the patent: **23.05.2018**
(21) Application number: 14701417.9
(22) Date of filing: 27.01.2014
(51) Int. Cl.: A61F 2/24, A61B 17/02

(54) **TEMPORARY ATRIUM SUPPORT DEVICE**
INSTRUMENT FÜR DIE VORÜBERGEHENDE STÜTZUNG DES VORHOFS
DISPOSITIF TEMPORAIRE POUR LE SUPPORT DE L'OREILLETTE

(30) Priority: 25.01.2013 EP 13152771; 25.01.2013 US 201361756663 P
(43) Date of publication of application: 02.12.2015
(73) Proprietor: Medtentia International Ltd Oy, 02600 Espoo (FI)
(72) Inventor: KERÄNEN, Olli, 237 41 Bjärred (SE); VIRTANEN, Jani, 01150 Sipoo (FI); PUGH, Mark, Coolaney, Co. Sligo (IE); O'CARROLL, Ger, Co. Sligo (IE); MORAN, Adrian, Co. Sligo (IE)
(86) International application number: PCT/EP2014/051542
(87) International publication number: WO 2014/114797

(56) References cited:
- EP-A1- 2 082 690
- WO-A1-2005/094729
- US-A1- 2007 066 993
- US-A1- 2008 027 268
- US-A1- 2011 257 461
- US-A1- 2011 307 003

## Description

### BACKGROUND OF THE INVENTION

### Related applications

This application is related to application EP13152770.7, US 61/756,649 "A valve for short time replacement, for taking over the function of and/or for temporary or partial support of a native valve in a heart" filed 25.01.2013, application EP13152769.9, US 61/756,657 "A medical system, and a device for collecting chordae and/or leaflets" filed 25.01 .2013, application EP13152774.9, US 61/756,633 "A medical device and method for facilitating selection of an annuloplasty implant" filed 25.01.2013 and application EP13152768.1, US 61/756,670 "A system for cardiac valve repair" filed 25.01.2013.

### Field of the Invention

This invention pertains in general to the field of medical devices for improvement of an atrium. More particularly the invention relates to a temporary atrium support device.

### Description of the Prior Art

During heart surgery at an atrium or related to the atrium, where the function of the atrium need to be or is reduced, there is an increased risk of the atrium collapsing. The collapse of the atrium may be induced by a variety of factors such as a pressure drop in the atrium, and/or a structural deficiency of the atrium. The pressure drop in the atrium may be due to a procedure of implanting a valve or other procedures resulting in a reduced pressure in the atrium.

A collapse of the atrium is highly undesired because it strongly influences the functioning of the heart and thus affects a patient in a non desired way. During surgery or intervention this means additional effort and personal is needed to maintain and monitor the atrium keeping the atrium functioning normally.

Hence, there is a strong need for preventing the collapse of the atrium to secure proper functioning of the heart and thus providing space for mitral intervention and the well being of a patient.

Prior art devices are insufficient in providing such functions.

US2007/0066993 discloses a method of preventing ingress of material into the left atrium of a heart that includes providing a delivery sheath, advancing the sheath distal end through an opening between the right atrium and the left atrium of the heart, providing an expandable cage, delivering the expandable cage to the left atrium, and expanding the expandable cage within the left atrium.

US 2011/0307003 discloses an implantable liner device for preventing formation of blood clots in a left atrium of a patient's heart.

US201 1257461 discloses a ventricular function assisting device configured to be implanted in a heart ventricle designed in a form of flower-like configuration comprising two or more petals attached at a base section.

US2008027268 discloses an apparatus implantable in a heart ventricle and that includes a frame configured to engage an inner circumferential periphery of the ventricle and to expand and contract between an expanded state corresponding to a desired end diastolic diameter of the ventricle and a contracted state corresponding to a desired end systolic diameter of the ventricle.

WO2005094729 discloses a reinforcing element used to structurally reinforce a portion of an endocardial surface of a heart.

EP2082690 discloses a medical device that assists diastolic function and prevents ventricular enlargement.

### SUMMARY OF THE INVENTION

Accordingly, examples of the present invention preferably seek to mitigate, alleviate or eliminate one or more deficiencies, disadvantages or issues in the art, such as the above-identified, by providing an temporary atrium support device according to the appended patent claims.

According to aspects of the disclosure, an atrium support device is disclosed.

According to a first aspect of the disclosure, a temporary atrium supporting device is provided. The atrium support device comprises an expandable and contractible intra-atrial support member being resiliently flexible to allow for atrium contraction and expansion, when positioned intra-atrial substantially maintaining atrial displacement volume of the beating heart. By using a temporary atrium support device a collapse of the atrium is prevented. The collapse of the atrium could be a consequence of an atrium pressure drop introduced by e.g. performing a repair of a mitral valve and/or other procedures performed in relation with the functioning of the atrium. The atrium support device thus secures the function of the atrium and consequently a heart during a medical procedure performed on the heart.

According to a second aspect of the disclosure not forming part of the claimed invention, a method of temporary preventing atrial collapse in a beating heart is provided. The method comprises intra-atrial positioning an expandable and contractible atrium support device, the atrium support device being resiliently flexible when expanded, and expanding the atrium support device in the atrium thus keeping the atrium non-collapsed, and allowing contraction and expansion of the non-collapsed atrium by the atrium support device.

Further examples are defined in the dependent claims, wherein features for the second and subsequent aspects of the disclosure are as for the first aspect mutatis mutandis.

Some examples of the disclosure provide for an atrium support member having a volume that never is smaller than a predefined volume.

Some examples of the disclosure provide for an atrium support member securing that there is a minimum of desired blood present in the atrium.

Some examples of the disclosure provide for a minimum blood flow through the heart is ensured.

Some examples of the disclosure provide for a beating heart and/or heart support equipment to maintain a minimum circulation of blood in a patient.

Some examples of the disclosure provide for a flexibility of an atrium support member to result in a maximum volume of the atrium support member.

Some examples of the disclosure provide an atrium to be controlled to result in a maximum atrium volume.

Some examples of the disclosure provide for aiding in an atriums reshaping to resume a maximum volume during relaxation of the atrium.

Some examples of the disclosure provide for reducing a damage of an atrium by over-expansion.

Some examples of the disclosure provide for an expansion of an atrium support member to be performed in a variety of ways.

Some examples of the disclosure provide for an atrium support device to be comprised of a wide selection of materials capable of being temporary introduced into the atrium.

Some examples of the disclosure provide for an atrium support device which does not induce any damages to the catheter and/or the atrium.

Some examples of the disclosure provide for expansion of an atrium support member in a controlled and faster way.

Some examples of the disclosure provide for better customization of an atrium support device to better suit a shape of an atrium. Embodiments of the invention provide for an atrium support device which easily is deployed trough a catheter.

Some examples of the disclosure provide for aligning and securing an atrium support device.

Some examples of the disclosure provide for an atrium support device remaining substantially at a same location during an expansion and a contraction of a heart.

Some examples of the disclosure provide for no unnecessary damages occurring due to any rotation and/or twisting of an atrium support member.

Some examples of the disclosure provide for blood flow to be secured between the pulmonary vein and the mitral valve.

Some examples of the disclosure provide for a simple but yet effective alignment of an atrium support device.

Some examples of the disclosure provide for partial support of an atrium.

Some examples of the disclosure provide for maximum support and prevention of collapse of an atrium.

Some examples of the disclosure provide for customization of the overall shape and size of an atrium support device to better adapt to a shape of an atrium.

Some examples of the disclosure provide for better compliance of an atrium support device with an expansion and contraction of the atrium during beating of a heart. Embodiments of the invention provide guiding means for deploying an annuloplasty device to a heart valve.

Some examples of the disclosure provide for faster attachment of an annuloplasty device.

Some examples of the disclosure provide for a simple yet effective solution for guiding an annuloplasty device into place.

Some examples of the disclosure provide for protection of tissue and/or other parts of an atrium when positioning an annuloplasty device.

Some examples of the disclosure provide for a minimum of strain to be exerted to an annuloplasty device when deployed at a desired heart location. Embodiments of the invention provide for a more accurate and simple guiding of an annuloplasty device to the desired heart site.

Some examples of the disclosure provide for a positioning of an atrium support device through an existing opening into an atrium.

Some examples of the disclosure provide for a positioning of an atrium support device to result in a minimum of damage of an atrium.

Some examples of the disclosure provide for a possibility to introduce other tools and/or devices through existing natural openings into an atrium.

Some examples of the disclosure provide for reducing any complications of a leak if an atrium support device into an atrium.

Some examples of the disclosure provide for accessible means during a heart surgery to inflate an atrium support member.

The meaning of atrial displacement in this application should be understood to mean the throughput of blood through the atrium and to provide space for mitral valve intervention.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings.
Fig. 1 is a cross sectional view of a heart with an expanded atrium support member;
Fig. 2 is a cross sectional view of a heart with an atrium support member positioned with a catheter through a mitral valve;
Fig. 3 is a cross sectional view of a heart with an expanded atrium support member deployed with a catheter through a mitral valve;
Fig. 4 is a cross sectional view of an atrium with an expanded atrium support member positioned through an aorta.
Fig. 5 is a perspective view of an embodiment of an expanded atrium support member comprising means for aligning;
Fig. 6 is a cross sectional view of an embodiment of an expanded atrium support member comprising a channel through the atrium support member from a pulmonary vein to a mitral valve;
Fig. 7 is a perspective view of an embodiment of an expanded atrium support member comprising guiding means at an outer surface.

### DESCRIPTION OF THE PREFERRED EXAMPLES

Specific examples of the disclosure will now be described with reference to the accompanying drawings. This invention may, however, be embodied in many different forms and should not be construed as limited to the examples set forth herein; rather, these examples are provided so that this disclosure will be thorough and complete, and will fully convey the scope of the invention as defined by the appended claims to those skilled in the art. The terminology used in the detailed description of the examples illustrated in the accompanying drawings is not intended to be limiting of the invention. In the drawings, like numbers refer to like elements.

The following description focuses on an example of the present disclosure applicable to a support device and in particular to a support device for an atrium. However, it will be appreciated that the disclosure is not limited to this application but may be applied to many other areas in a body where support is needed.

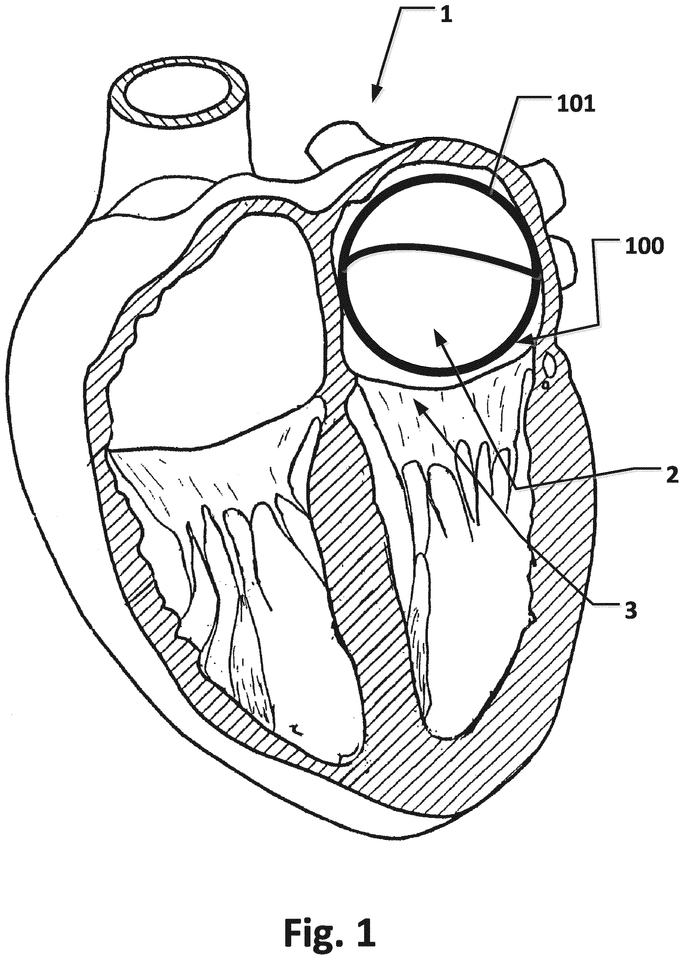

In an example of the disclosure according to Fig. 1, a temporary expandable and contractible atrium supporting device 100 positioned inside an atrium 2 in a heart 1 is shown. The atrium supporting device 100 comprises an expandable and contractible intra atrial support member 101. The support member 101 is resiliently flexible to allow for atrium 2 contraction and expansion, when positioned intra atrial. The support member 101 substantially maintains atrial displacement volume of the beating heart. By using a temporary atrium support device 100 a collapse of the atrium 2 is prevented. The collapse of the atrium 2 could be a consequence of a pressure drop in the atrium 2 introduced by e.g. when performing a repair of a mitral valve 3 and/or other procedures performed in relation with the functioning of the atrium 2. The atrium support device 100 thus secures the function of the atrium 2 and consequently the heart during a procedure which is related to the atrium 2 and/or the atrium 2 itself. Further, by being resiliently flexible to allow for natural atrium contraction and expansion while maintaining an atrial displacement volume, disturbance of the heart function is minimized. Other devices, such as those placed in the atrium for occlusion of defects, or re-shaping devices, or other devices for permanent anchoring provide too high force to the anatomy, or permanent damage to the tissue, due to their different purpose, and consequently disturb the natural heart function significantly. Also, devices for lining or coating portions of the heart to prevent blood cloths does not provide any support for the atrium to maintain a displacement volume of a collapsing atrium. On contrary, the atrium support member of the present disclosure improve the compatibility and synchronous function with the heart due to being resiliently flexible its expanded state to follow the natural beating movement.

In order to maintain a normal or an adequate function of the atrium 2 the resilient flexibility of the atrium support member 101 results in an atrium volume that is preferably more than 55ml, more preferably more than 50ml, even more preferably more than 20ml and most preferably more than 15ml. By having the atrium support member 101 resiliently flexible such that the atrium support member 101 has a volume that may be changed and that never is smaller than a predefined volume, the atrium support member 101 secures that there is a minimum of desired blood present in the atrium 2 and a minimum blood flow is thus ensured. This allows for a beating heart and/or heart support equipment to maintain a minimum circulation of blood in a patient. Further, the resilient flexibility of the atrium support member 101 results in an atrium volume that is preferably at the most 100ml, more preferably at the most 90ml, even more preferably at the most 80ml and most preferably at the most 60ml. By allowing the resiliently flexibility of the atrium support member 101 to obtain more than a maximum volume of the atrium 2 the atrium 2 is controlled to result in a maximum atrium volume. Additionally, by defining the flexibility of the atrium support member 101 to the maximum volume the atrium 2 is aided in its reshaping to resume the maximum volume during relaxation of the atrium 2. Further, by constraining the flexibility of the atrium support member 101 to the maximum volume, damage of the atrium 2 by over-expansion is reduced.

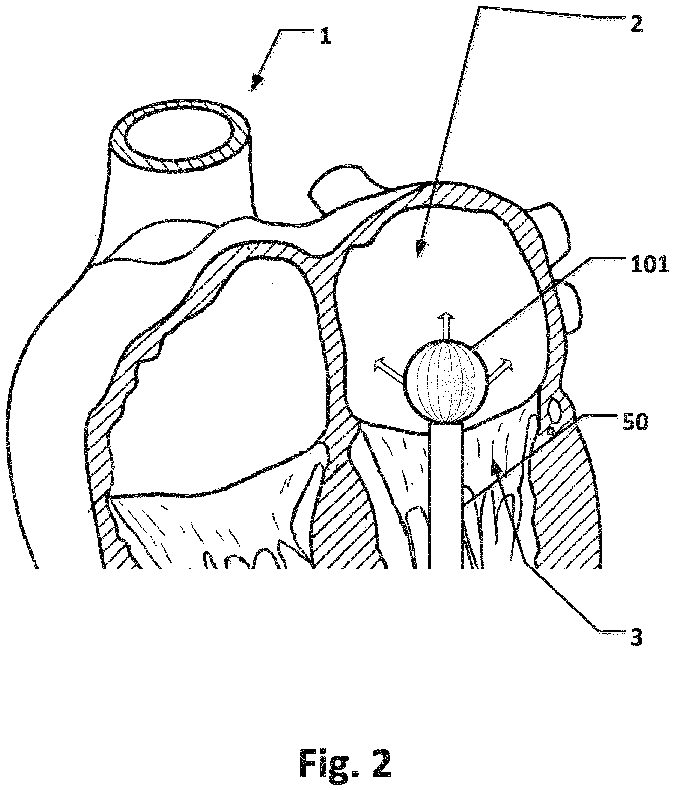

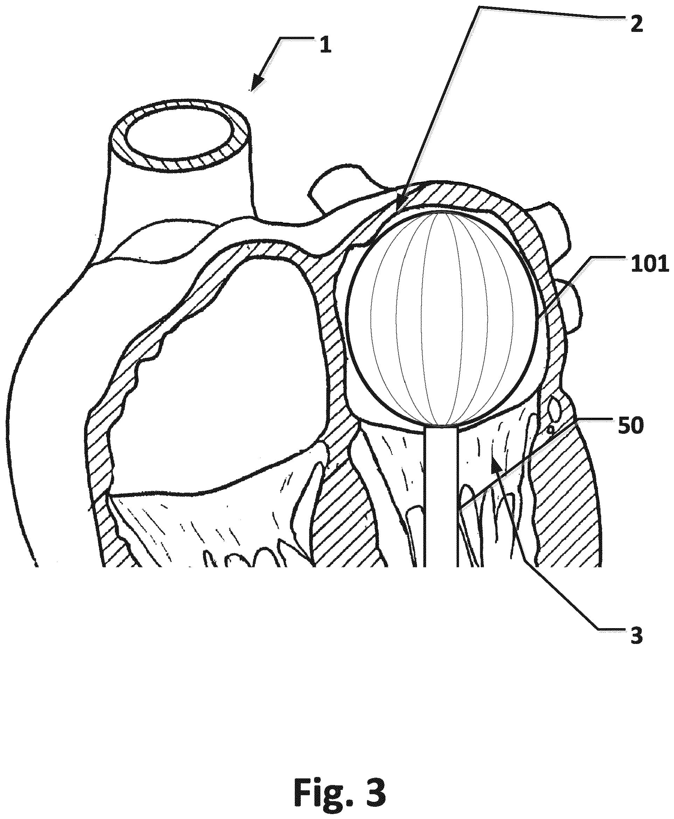

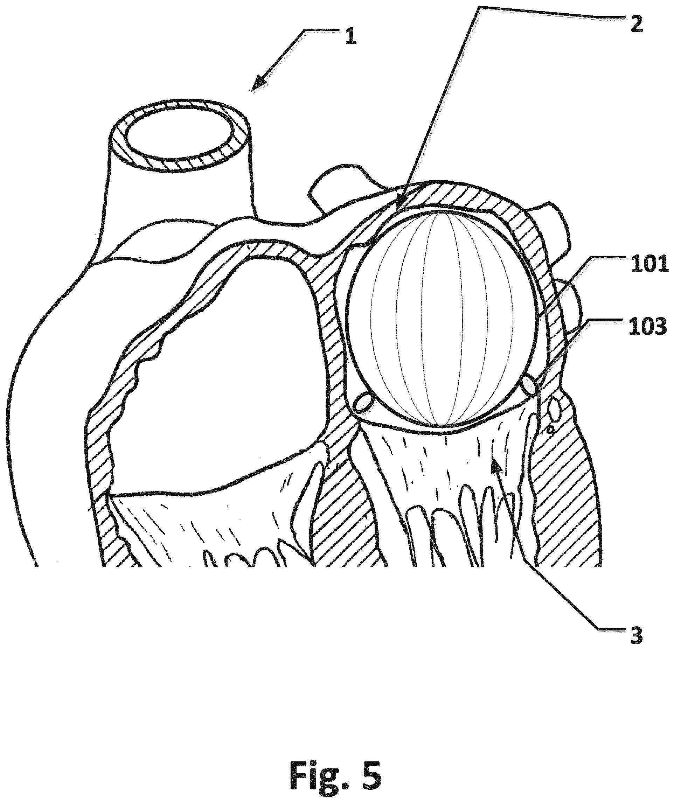

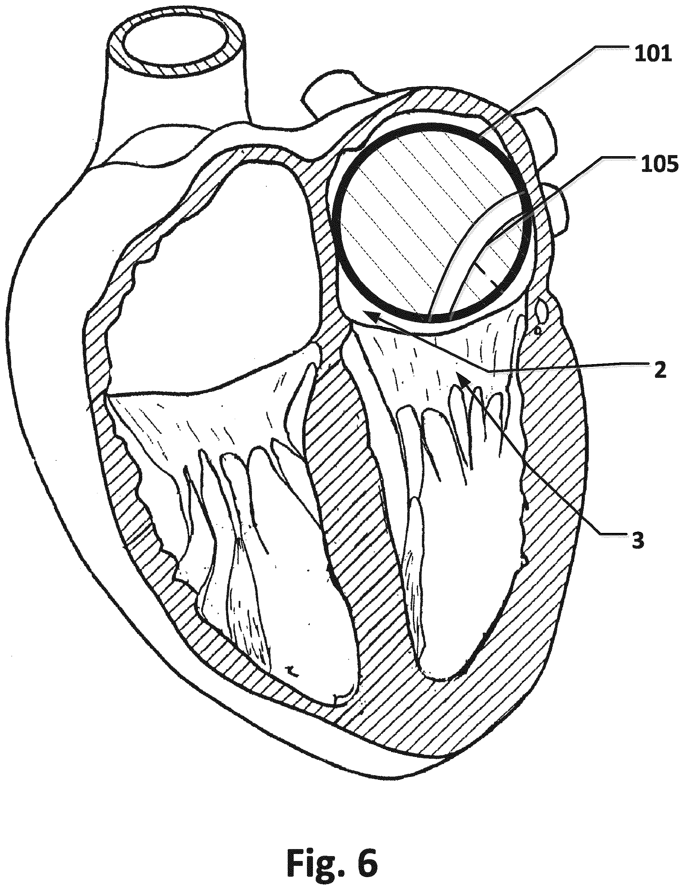

The atrium support member may thus have a predefined maximum expanded cross-section. The atrium support member may thus also have a predefined minimum contracted cross-section when placed in the atrium, such that the compressive force exerted by the atrium on the atrium support member at the minimum contracted cross-section is compensated and counter acted by a reaction force of the support member on the atrium, and where the reaction force is equal to that of said compressive force. The reaction force at the minimum contracted cross-section can thus be set to a pre-defined value. This can e.g. be done during heat setting procedures of the material to define its properties.
The atrium support member 101 is of a shape and material that is capable of being inserted and guided through a catheter 50 to the atrium 2 as illustrated in Figs. 2-4. The atrium support member 101 may comprise an expandable cage, as illustrated in Figs. 1-3, 5 and 7, and/or alternatively a wire, and/or an inflatable member comprising at least one channel 105, as illustrated in Fig. 6, and/or a covering comprising holes which allows for substantially maintaining the atrial displacement volume. In one example the expandable cage comprises at least two intersecting cage rings, but more preferably more than two intersecting cage rings in order to achieve more area of contact between the cage rings outer surface towards the atrium tissue and the atrium tissue. The atrium support member may comprise an inlet and outlet for blood flow. This assures that the natural flow of blood is not disturbed while maintaining the displacement volume.
In another example the at least one channel in the inflatable member comprises a valve. The valve may be of the cardiac valve type, i.e. being closed when the heart chamber ejects blood to the body or lungs respectively and open during a refill phase. The dimension of the at least one channel and any possible valve is chosen based on a wanted atrial displacement. The same applies for selection of the size of the holes in the covering.

The expansion of the atrium support member 101 may be performed in a variety of ways and the atrium support device 100 may be comprised of a wide selection of materials capable of being temporary introduced into the atrium 2 and being expandable from the catheter 50 without diverging from the scope of the invention. Following, some examples will be giving but they should not be construed as limiting.

The atrium support member 101 is preferably made of a material that is biocompatible and designed in such way that the atrium support member 101 does not induce any damages to the catheter 50 and/or the atrium 2.

For example the atrium support device 100 comprises a memory shape material, wherein the memory shape material has a first shape when deployed and a second expanded shape activated by a shape memory temperature. By using the atrium support device 100 comprising the shape memory material it is possible to activate the atrium support device 100 to expand to the second expanded shape in a controlled and faster way than with other expansion techniques. Further, the memory shape material allows for better customising the expanded shape of the atrium support device 100 to better suit the shape of the atrium 2. Suitable materials for the shape memory material are e.g. copper-aluminium-nickel alloys, nickel-titanium alloys and/or other known shape memory materials.

In yet another example the atrium support member 101 comprises a heat set shape, and wherein the atrium support member 101 elastically returns to the heat set shape. By use of the heat set shape it is possible to get an atrium support member 101 which has inherent elastic properties to a desired shape and can easily be deployed trough the catheter 50. Suitable materials for the heat set shape are e.g. nickel-titanium alloys and/or other known heat set shape materials.

In another example, the atrium support device 100 comprises and/or acts as, a leaflet limiter which limits abnormal movement, such as prolapse, of the leaflets into the atrium. Such abnormal movement may arise if a chordae, or several chordae, that usually limits the movement of the leaflet is completely destroyed and the leaflet may thus freely move in the left atrium and/or left chamber. The leaflet limiter is made of a material that can expand together with the atrium support device 100, and/or it may be made of the same material as the atrium support device 100. Alternatively, the leaflet limiter is expanded by a spring back when exited from the catheter 50. The leaflet limiter may be a crossbar that extends and is projected laterally from the atrium support device 100. The number of leaflet limiters and their placement is chosen based on the circumstance that the atrium support device 100 is used in and may thus be of a number of different shapes and have various placements. One example would be to have a simple projection outwards towards the leaflets from the atrium support device 100 that limits the movement or other suitable shapes that limits but not damage the leaflet(s) when hindering its movement into the atrium. Preferably, the atrium support device 100 has two leaflet limiters, one on each side of the atrium support device 100 for each leaflet. But, there could also be only one leaflet limiter if it is known that one leaflet is already damaged and moving freely when starting a procedure of deploying the atrium support device 100. As mentioned, the atrium support device itself may acts as a leaflet limiter when expanded in the atrium, and no other component may be required. This provides for a device that limits leaflet movement that is easy to handle and position.

In an example the atrium support device 100 comprises means for aligning 103 the atrium support device 100 by use of at least one commissure, wherein the means for aligning 103 comprises a first end and a second end, and wherein the first end of the means for aligning is connected to the atrium support member 101 and the second end is directed outwards from the atrium support member 101 towards the at least one commissure. The means for aligning the atrium support member 101 assists in aligning and securing the atrium support member 101 from rotation when the atrium 2 is e.g. relaxed with a larger volume than the atrium support member 101 and/or the atrium support member 101 comprises the inflatable member with the at least one channel 105. In one example, if the atrium 2 is relaxed with the larger volume than the atrium support member 101 the means for aligning is for securing and aligning the atrium support member 101 to the atrium 2 so that the atrium support member 101 remains substantially at the same location during the expansion and contraction of the heart and no unnecessary damages occurs due to any rotation and/or twisting of the atrium support member 101. In another example, if the atrium support member 101 comprises the inflatable member with at least one channel 105 which extends from the pulmonary vein to the mitral valve 3, the means for anchoring ensures that the atrium support member 101 aligns the channel 105 to the pulmonary vein and the mitral valve 3 during the expansion and contraction of the atrium 2 so that blood flow is secured between the pulmonary vein and the mitral valve 3.

The means for aligning 103 the atrium support member 101 is in one example a pair of projections 103, projecting outwards from the atrium support member 101. By using a pair of projections 103 for aligning the atrium support member 101, a simple but yet effective alignment of the device is achieved. For example the atrium support member 101 is easily aligned by simply using the pair of projections 103 at and/or into suitable aligning sites in the atrium 2 such as the commissures and/or the mitral valve 3 and the pulmonary vein.

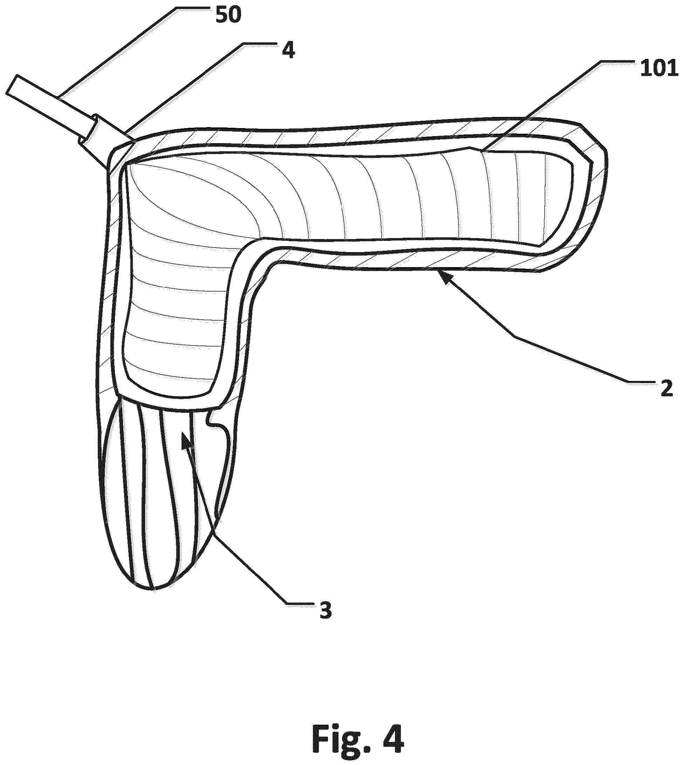

In some examples the atrium support member 101 is partly in contact with the atrium 2. By allowing the atrium support device 100 being partly in contact with the atrium 2 the support device is e.g. chosen to support a larger section of the atrium 2 or chosen to support a smaller section of the atrium 2. Alternatively, the atrium support member 101 is in contact with substantially the entire atrium 2 as illustrated in Fig. 4. By having the support device contacting the entire atrium 2, maximum support and prevention of collapse of the atrium 2 is ensured.

The atrium support member 101 may be designed in a variety of ways to provide partial contact or substantially full contact or get into apposition with the atrium 2. Such designs are e.g. the atrium support member 101 is bent when expanded, such as banana shaped as for instance in Fig. 4. The atrium support member 101 may be spherical when expanded. The atrium support member 101 may be bulb shaped when expanded. Alternatively, the atrium support device 100 comprises a plurality of atrium support member 101. By use of a plurality of atrium support members 101 it is possible to customise the overall shape and size of the atrium support device 100 to better adapt to the shape of the atrium 2 than a single atrium support member 101. Further, it would be possible to have atrium support members 101 with different flexibility at different locations in the atrium 2 which allows for better compliance of the atrium support device 100 with the expansion and contraction of the atrium 2 during beating of the heart. In embodiments according to the invention, the atrium support device 100 further comprises means for guiding 107 an annuloplasty device to a securing site at a heart valve. By having the atrium support device 100 further comprising guiding means 107 it is possible to aid the operator to in deploying the annuloplasty device to the heart valve. This allows for faster attachment of the annuloplasty device compared to when the operator need to introduce other equipment to secure the annuloplasty device.

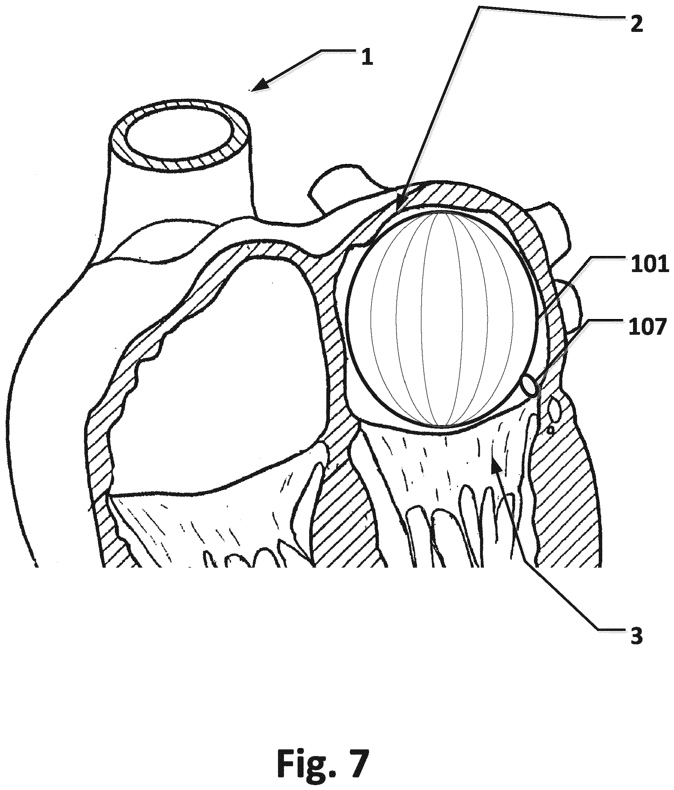

In one embodiment the guiding means of the atrium support device 100 is at least one ring shaped member 107 arranged at an outer surface of the atrium support member 101 which is illustrated in Fig. 7. The use of at least one ring shaped member 107 provides a simple yet effective solution for guiding the annuloplasty device into place. The outer surface of the atrium support member 101 is the surface facing the tissue of the atrium 2. The arrangement of the at least one ring shaped member 107 on the outer surface of the atrium support member 101 is achieved by e.g. attaching the ring shaped member 107 at, on or through the outer surface.

In another embodiment the means for guiding is a channel arranged along an outer surface of the atrium support member 101. The use of the channel as the guiding means is particularly beneficial when the atrium support member 101 is a sheet, covering or other shell shaped atrium support member 101. In such case the annuloplasty device is guided along the channel arranged along the outer surface of the atrium support device 100 to a desired heart site. The channel is preferably a coherent channel but may also be a sectioned channel.

In yet another variant not forming part of the claimed invention, the means for guiding is a plurality of holes at the atrium support member 101. The use of holes in the atrium support member 101 the guiding of the annuloplasty device is performed through the atrium support member 101 which allows for the atrium support member 101 to protect the tissue and/or other parts of the atrium 2 when positioning the annuloplasty device. Additionally, the use of the plurality of holes provides guiding when the annuloplasty device has a smaller diameter than the atrium support member 101 and a minimum of strain is to be exerted to the annuloplasty device.

In yet an alternative embodiment, the atrium support member 101 comprises e.g. the inflatable member or another substantially solid object, the means for guiding is at least one channel arranged through the atrium support member 101. The use of at least one channel through the atrium support member 101 provides the same solution for protecting the atrium 2 and exerting the minimum of strain as with the plurality of holes. In addition, the at least one channel through the atrium support member 101 ensures for a more accurate and simple guiding of the annuloplasty device to the desired heart site.

A further example of the disclosure is illustrated in Figs. 2-4 where a method of temporary preventing atrial collapse in a beating heart, comprising intra atrial positioning an expandable and contractible atrium support device 100, the atrium support device 100 being resiliently flexible when expanded and expanding the atrium support device 100 intra atrial thus keeping the atrium 2 non-collapsed and allowing contraction and expansion of the non-collapsed atrium 2 by the atrium support device 100.

As illustrated in Figs. 2-4 an example of the positioning of the atrium support device 100 is performed through an existing opening into the atrium 2. In one example the positioning is performed through the mitral valve 3 as illustrated in Figs. 2-3, but other existing openings such as a pulmonary vein or an aorta 4, as illustrated in Fig. 4, can be used to introduce the atrium support device 100 into the atrium 2. By using the existing openings to the atrium 2 a minimum of damage of the atrium 2 is achieved. Well known procedures using these openings are transapically through the mitral valve 3 or trans-femorally through the aortic valve and the mitral valve 3 for entering the left atrium.

Alternatively in another example, the positioning of the atrium support device 100 is performed through an atrium wall. By having the atrium support device 100 positioned through the atrium wall it is possible to introduce other tools and/or devices through the existing openings. The existing openings may also be blocked and/or to narrow to introduce the atrium support device 100 resulting in that the operator is forced to go through the atrium wall and/or septum wall.

The expansion of the atrium support device 100 intra-atrial preventing the collapse of the atrium 2 is preferably performed by use of a force on the atrium support device 100. Such force may be a pulling force, a pushing force, an elastic force and/or an expansion force.

In another example the expansion of the atrium support device 100 is performed by use of applying a shape memory temperature to the atrium support device 100. By using the shape memory temperature to the atrium support device 100 the atrium support device is triggered to expand. The shape memory temperature is chosen to be triggered at e.g. a temperature of the blood in the atrium 2 or a temperature of a heating element.

In yet another example the expansion of the atrium support device 100 is performed by use of supplying a gas or liquid to the atrium support device 100. The use of the liquid for expanding the atrium support device 100 allows for water or blood to inflate the atrium support device 100 comprising an inflatable member reducing any complications if a leak of the atrium support device 100 would occur. If using gas to inflate the atrium support device 100 comprising the inflatable member the atrium support device 100 is inflated by using accessible means during heart surgery.

The present invention has been described above with reference to specific embodiments. However, other embodiments than the above described are equally possible within the scope of the invention. The scope of the invention is only limited by the appended patent claims.

## Claims

1. A temporary atrium support device (100) for delivery with a catheter (50), comprising:
an expandable and contractible intra atrial support member (101) being resiliently flexible to allow for atrium contraction and expansion, when positioned intra atrial substantially maintaining atrial displacement volume of the beating heart,
**characterized by** means for guiding (107) an annuloplasty device to a securing site at a heart valve, wherein said means for guiding (107) is one of:
- at least one ring shaped member (107) attached to an outer surface of the atrium support member,
- a channel arranged along an outer surface of the atrium support member, or
- at least one channel (105) arranged through the atrium support member from an entry point on the surface of the support member to an exit point on the surface of the support member.

2. The atrium support device according to any of the preceding claims, wherein the atrium support device (100) comprises a plurality of atrium support members.

3. The atrium support device according to any of the preceding claims, wherein the atrium support member (101) is an expandable cage, a wire, an inflatable member comprising at least one channel and/or an expandable covering with openings which allows for substantially maintaining the atrial displacement volume.

4. The atrium support device according to any of the preceding claims, wherein the atrium support member (101) comprises an inlet and outlet for blood flow.

5. The atrium support device according to any of the preceding claims, wherein the atrium support member (101) comprises a memory shape material, wherein the memory shape material has a first shape when deployed and a second expanded shape activated by a shape memory temperature.

6. The atrium support device according to any of the preceding claims, wherein the atrium support member (101) comprises a heat set shape, and
wherein the atrium support member (101) elastically returns to the heat set shape.

7. The atrium support device according to any of the preceding claims, further comprises means for aligning (103) the atrium support device (100) by use of at least one commissure, wherein the means for aligning (103) comprises a first end and a second end, and
wherein the first end of the means for aligning (103) is connected to the atrium support member (101) and the second end is directed outwards from the atrium support member (101) towards the at least one commisure.

8. The atrium support device according to claim 7, wherein the means for aligning (103) are a pair of projections, projecting outwards from the atrium support member (101).

9. The atrium support device according to any of the preceding claims, wherein the atrium support member (101) is configured to be partly in contact with the atrium.

10. The atrium support device according to any of the preceding claims, wherein the atrium support member (101) is configured to be in contact with substantially the entire atrium.

11. The atrium support device according to any of the preceding claims, wherein the atrium support member (101) is bent, banana shaped, spherical and/or bulb shaped when expanded.

12. The atrium support device according to any of the preceding claims, wherein said atrium support member (101) is resiliently flexible in an expanded state of the atrium support member (101).

13. The atrium support device according to any of the preceding claims, wherein the atrium support member (101) has a predefined maximum expanded cross-section.

14. The atrium support device according to any of the preceding claims, wherein the atrium support member (101) has a predefined minimum contracted cross-section when placed in the atrium, such that the compressive force exerted by the atrium on the atrium support member (101) at the minimum contracted cross-section is compensated and counter acted by a reaction force of the support member (101) on the atrium, the reaction force being equal to that of said compressive force, wherein said reaction force at the minimum contracted cross-section is set to a pre-defined value.

## Patentansprüche

1. Vorrichtung (100) für die vorübergehende Stützung des Vorhofs zur Freisetzung mit einem Katheter (50), umfassend:
ein expandierbares und kontrahierbares intraatriales Stützelement (101), das federnd flexibel ist, um Kontraktion und Expansion des Vorhofs zu erlauben, wobei es, wenn es intraatrial angeordnet ist, das atriale Verschiebevolumen des schlagenden Herzens im Wesentlichen aufrechterhält,
**gekennzeichnet durch** Mittel (107) zum Führen einer Annuloplastie-Vorrichtung zu einer Befestigungsstelle an einer Herzklappe, wobei das Führungsmittel (107) eines der folgenden ist:
- mindestens ein ringförmiges Element (107), das an einer Außenfläche des Vorhofstützelements befestigt ist,
- ein Kanal, der entlang einer Außenfläche des Vorhofstützelements angeordnet ist, oder
- mindestens ein Kanal (105), der durch das Vorhofstützelement von einem Eintrittspunkt auf der Oberfläche des Stützelements bis zu einem Austrittspunkt auf der Oberfläche des Stützelements angeordnet ist.

2. Vorhofstützvorrichtung nach einem der vorhergehenden Ansprüche, wobei die Vorhofstützvorrichtung (100) eine Vielzahl von Vorhofstützelementen umfasst.

3. Vorhofstützvorrichtung nach einem der vorhergehenden Ansprüche, wobei das Vorhofstützelement (101) ein expandierbarer Käfig, ein Draht, ein aufblasbares Element, das mindestens einen Kanal umfasst, und/oder eine expandierbare Abdeckung mit Öffnungen ist, der bzw. das bzw. es erlaubt, das atriale Verschiebevolumen im Wesentlichen aufrechtzuerhalten.

4. Vorhofstützvorrichtung nach einem der vorhergehenden Ansprüche, wobei das Vorhofstützelement (101) einen Einlass und einen Auslass für Blutfluss umfasst.

5. Vorhofstützvorrichtung nach einem der vorhergehenden Ansprüche, wobei das Vorhofstützelement (101) ein Formgedächtnismaterial umfasst, wobei das Formgedächtnismaterial eine erste Form bei der Freisetzung und eine zweite expandierte Form, die durch eine Formgedächtnistemperatur aktiviert wird, aufweist.

6. Vorhofstützvorrichtung nach einem der vorhergehenden Ansprüche, wobei das Vorhofstützelement (101) eine durch Wärme gehärtete Form umfasst, und wobei das Vorhofstützelement (101) elastisch in seine durch Wärme gehärtete Form zurückkehrt.

7. Vorhofstützvorrichtung nach einem der vorhergehenden Ansprüche, ferner umfassend Mittel (103) zum Ausrichten der Vorhofstützvorrichtung (100) durch Verwendung mindestens einer Kommissur, wobei das Ausrichtungsmittel (103) ein erstes Ende und ein zweites Ende umfasst, und
wobei das erste Ende des Ausrichtungsmittels (103) mit dem Vorhofstützelement (101) verbunden ist und das zweite Ende vom Vorhofstützelement (101) nach außen zu der mindestens einen Kommissur gerichtet ist.

8. Vorhofstützvorrichtung nach Anspruch 7, wobei die Ausrichtungsmittel (103) zwei Vorsprünge sind, die vom Vorhofstützelement (101) nach außen ragen.

9. Vorhofstützvorrichtung nach einem der vorhergehenden Ansprüche, wobei das Vorhofstützelement (101) dazu ausgelegt ist, teilweise mit dem Vorhof in Kontakt zu sein.

10. Vorhofstützvorrichtung nach einem der vorhergehenden Ansprüche, wobei das Vorhofstützelement (101) dazu ausgelegt ist, im Wesentlichen mit dem gesamten Vorhof in Kontakt zu sein.

11. Vorhofstützvorrichtung nach einem der vorhergehenden Ansprüche, wobei das Vorhofstützelement (101) gebogen, bananenförmig, kugelförmig und/oder knollenförmig ist, wenn es expandiert ist.

12. Vorhofstützvorrichtung nach einem der vorhergehenden Ansprüche, wobei das Vorhofstützelement (101) in einem expandierten Zustand des Vorhofstützelements (101) federnd flexibel ist.

13. Vorhofstützvorrichtung nach einem der vorhergehenden Ansprüche, wobei das Vorhofstützelement (101) einen vorbestimmten maximalen expandierten Querschnitt aufweist.

14. Vorhofstützvorrichtung nach einem der vorhergehenden Ansprüche, wobei das Vorhofstützelement (101) einen vorbestimmten kontrahierten Mindestquerschnitt aufweist, wenn es im Vorhof platziert ist, so dass die vom Vorhof auf das Vorhofstützelement (101) ausgeübte Kompressionskraft beim kontrahierten Mindestquerschnitt durch eine Reaktionskraft des Stützelements (101) auf den Vorhof kompensiert und ihr entgegengewirkt wird, wobei die Reaktionskraft der Kompressionskraft gleicht, wobei die Reaktionskraft beim kontrahierten Mindestquerschnitt auf einen vorbestimmten Wert eingestellt wird.

## Revendications

1. Dispositif de support auriculaire temporaire (100) pour placement avec un cathéter (50), comprenant :
un élément de support intra-auriculaire extensible et contractile (101) étant élastiquement flexible pour permettre la contraction et la dilatation de l'oreillette, lorsqu'il est positionné de façon intra-auriculaire en maintenant le volume de déplacement auriculaire du coeur battant, **caractérisé par** un moyen de guidage (107) d'un dispositif d'annuloplastie vers un site de fixation au niveau d'une valvule cardiaque, dans lequel ledit moyen de guidage (107) est l'un de :
- au moins un élément de forme annulaire (107) fixé à une surface externe de l'élément de support auriculaire,
- un canal agencé le long d'une surface externe de l'élément de support auriculaire, ou
- au moins un canal (105) agencé à travers l'élément de support auriculaire d'un point d'entrée sur la surface de l'élément de support à un point de sortie sur la surface de l'élément de support.

2. Dispositif de support auriculaire selon l'une quelconque des revendications précédentes, le dispositif de support auriculaire (100) comprenant une pluralité d'éléments de support auriculaire.

3. Dispositif de support auriculaire selon l'une quelconque des revendications précédentes, dans lequel l'élément de support auriculaire (101) est une cage extensible, un fil métallique, un élément gonflable comprenant au moins un canal et/ou une couverture extensible avec des ouvertures qui permet de maintenir sensiblement le volume de déplacement auriculaire.

4. Dispositif de support auriculaire selon l'une quelconque des revendications précédentes, dans lequel l'élément de support auriculaire (101) comprend une entrée et une sortie pour l'écoulement de sang.

5. Dispositif de support auriculaire selon l'une quelconque des revendications précédentes, dans lequel l'élément de support auriculaire (101) comprend un matériau à mémoire de forme, dans lequel le matériau à mémoire de forme a une première forme lorsqu'il est déployé et une deuxième forme dilatée lorsqu'il est activé par une température de mémoire de forme.

6. Dispositif de support auriculaire selon l'une quelconque des revendications précédentes, dans lequel l'élément de support auriculaire (101) comprend une forme thermodurcie, et
dans lequel l'élément de support auriculaire (101) retourne élastiquement à la forme thermodurcie.

7. Dispositif de support auriculaire selon l'une quelconque des revendications précédentes, comprenant en outre un moyen d'alignement (103) du dispositif de support auriculaire (100) par utilisation d'au moins une commissure, dans lequel le moyen d'alignement (103) comprend une première extrémité et une deuxième extrémité, et
dans lequel la première extrémité du moyen d'alignement (103) est raccordée à l'élément de support auriculaire (101) et la deuxième extrémité est dirigée vers l'extérieur depuis l'élément de support auriculaire (101) vers l'au moins une commissure.

8. Dispositif de support auriculaire selon la revendication 7, dans lequel le moyen d'alignement (103) sont une paire de saillies, faisant saillie vers l'extérieur depuis l'élément de support auriculaire (101) .

9. Dispositif de support auriculaire selon l'une quelconque des revendications précédentes, dans lequel l'élément de support auriculaire (101) est configuré de façon à être partiellement en contact avec l'oreillette.

10. Dispositif de support auriculaire selon l'une quelconque des revendications précédentes, dans lequel l'élément de support auriculaire (101) est configuré pour être en contact sensiblement avec l'oreillette entière.

11. Dispositif de support auriculaire selon l'une quelconque des revendications précédentes, dans lequel l'élément de support auriculaire (101) est plié, en forme de banane, sphérique et/ou n forme d'ampoule lorsqu'il est dilaté.

12. Dispositif de support auriculaire selon l'une quelconque des revendications précédentes, dans lequel ledit élément de support auriculaire (101) est élastiquement flexible dans un état dilaté de l'élément de support auriculaire (101).

13. Dispositif de support auriculaire selon l'une quelconque des revendications précédentes, dans lequel l'élément de support auriculaire (101) a une section transversale dilatée maximale prédéfinie.

14. Dispositif de support auriculaire selon l'une quelconque des revendications précédentes, dans lequel l'élément de support auriculaire (101) a une section transversale contractée minimale prédéfinie lorsqu'il est placé dans l'oreillette, de sorte que la force de compression exercée par l'oreillette sur l'élément de support auriculaire (101) à la section transversale contractée minimale soit compensée et contrebalancée par une force de réaction de l'élément de support (101) sur l'oreillette, la force de réaction étant égale à celle de ladite force de compression, dans lequel ladite force de réaction à la section transversale contractée minimale est ajustée à une valeur prédéfinie.
